# EUROPEAN PATENT APPLICATION

(11) **EP 3 892 982 A1**
(43) Date of publication of application: **13.10.2021**
(21) Application number: 20168719.1
(22) Date of filing: 08.04.2020
(51) Int. Cl.: G01N 21/03, B01L 3/00, G01N 21/359, G01N 33/49

(54) **CARTRIDGE FOR SPECTROSCOPIC DEVICE FOR QUANTIFICATION IN BIOLOGICAL FLUIDS, RESPECTIVE KIT AND METHOD OF USE THEREOF**

(71) Applicant: Gazellepodium - Lda, 4935 - 054 Darque (PT)
(72) Inventor: CARVALHO DE SOUSA, Nuno Jorge, 4200-139 Porto (PT)
(74) Representative: Maikowski & Ninnemann Patentanwälte Partnerschaft mbB

(57) **Abstract**

Cartridge for a spectroscopic, e.g. near-infrared, NIR, device for analyte quantification in a sample of a biological fluid, comprising a multilayered product comprising a paper-based layer (3) having porosity for absorbing and spreading the sample in substantially all of a predetermined area at a front side of the paper-based layer for the spectroscopic device to measure. The paper-based layer may comprise a NIR diffusely-reflecting paper material which provides a uniform optical path across said predetermined area. The multilayered product may comprise a reflective layer (4) for reflecting NIR light and backing the paper-based layer. The multilayered product may further comprise a back-support layer (5) to stabilise the paper-based layer and, if existing, the reflective layer. The multilayered product may further comprise a protective layer (2) of the front side of the paper-based layer, said protective layer being hydrophobic and having an opening for containing the absorbing and spreading of the sample to the predetermined area.

## Description

### TECHNICAL FIELD

The present disclosure relates to a disposable multilayer paper-based cartridge to be used with a spectroscopic device for quantification of parameters in biological fluids. In particular, the disclosure pertains to a near-infrared spectroscopic device for quantification of parameters in biological fluids comprising a disposable multilayer paper-based cartridge. The fluids may be blood, serum, plasma, urine or cerebrospinal fluid. The parameters include hemoglobin concentration, and the erythrocyte, haematocrit and RDW count.

### BACKGROUND

There are technologies proven to be effective for quantification of parameters in biological fluids, for example the Siemens ADVIA 120 Hematology System (tm). However, the ADVIA 120 Hematology System (which can be considered, among others, as a gold reference standard) is expensive, immobile, bulky, requires 175 µL of sample volume (venipuncture collection method), reagents and consumables, trained health professionals and it is not easily applied to point-of-care settings (outside of hospital conditions), namely: home, emergency situations, military scenarios or even in underdeveloped countries with lack of medical infrastructures.

There are portable solutions, as for example HemoCue (tm), that measure hemoglobin concentration, but it is a hemoglobin specific test. In contrast, the present disclosure is able to measure several parameters with the same blood sample.

These facts are disclosed in order to illustrate the technical problem addressed by the present disclosure.

### GENERAL DESCRIPTION

The present disclosure relates to a disposable multilayer paper-based cartridge to be used with a near-infrared spectroscopic device for quantification of parameters in biological fluids. In particular, the disclosure pertains to a near-infrared spectroscopic device for quantification of parameters in biological fluids comprising a disposable multilayer paper-based cartridge. The fluids may be blood, serum, plasma, urine or cerebrospinal fluid. The parameters include hemoglobin concentration, and the erythrocytes, hematocrit and RWD count.

The present disclosure comprises the use of a portable, near-infrared spectroscopic (NIR) sensor device combined with a disposable multilayer paper-based cartridge for the quantification of several parameters in biological fluids such as blood, serum, plasma, urine, cerebrospinal fluid. The disclosed device measures in blood the following parameters: hemoglobin concentration, and the erythrocytes, hematocrit and RDW count. Blood sample is collected and preferably homogeneously spread in the cartridge for spectroscopic analysis. Light absorbance spectra combined with chemometric methods and algorithms developed for each analyte allow for analyte prediction. NIR wavelengths can be for example 900-2500 nm. The disclosure is also applicable to a visible light VIS and near-infrared NIR (for example, wavelengths of 400-2500). Depending on the specific analyte and biological fluid, ultraviolet light UV may also be used (for example wavelengths 200-2500 nm).

The platform is portable, non-invasive and is able to produce results that require no operator calibration, interpretation or calculations. Therefore, the disclosure can be applied in point-of-care settings such as emergency situations, military scenarios, pharmacies and/or home.

One of the problems addressed by the disclosure concerns the quantification of key parameters in medical diagnosis by counting cells and by measuring the concentration of hemoglobin in a given volume of blood. These analyses are currently performed by expensive, high-volume equipment that works with a minimum sample volume of for example 175 µL thus requiring an invasive venipuncture collection procedure to collect the sample. Because it is a fixed and stationary equipment, it needs to be restricted in the laboratory and requires qualified health professionals to interact with the machine in several steps of sample flow.

The present disclosure differs, among other reasons, by its portability: the sensor is portable, and the cartridges are portable and disposable. The platform does not require other reagents or consumables and is prepared to analyze cellular components of the blood as well as metabolites thereof. The sample volume required does not exceed 40 µL, preferably does not exceed 30 µl, even more preferably does not exceed 20 µl. This volume can preferably be reduced to 10 µL by using different alpha layers of the cartridge, explained in more detail below, enhancing sample collection via finger prick.

The present disclosure is developed and applied for venous blood-plasma samples and capillary blood samples. The procedure for analysis requires the placement of the blood sample in the cartridge region with a waiting period, preferably for the sample to completely spread. This waiting period should not exceed 60 seconds. The waiting period is preferably in a time range between 30 and 60 seconds. Time is a fixed variable and it is crucial for system performance that waiting period never exceeds 60 seconds.

Later, the user should preferably perform a sealing process, sliding the omega layer explained in more detail below. Then, the cartridge should be placed in a suitable NIR support for near-infrared spectroscopic analysis. The platform does not require user intervention during the analysis steps and produces real-time, automatic results that do not require interpretation or calculations. The generated results were validated against the gold standard technology and proven to be an effective and alternative method, particularly with respect to the Siemens ADVIA 120 Hematology System.

The present disclosure includes the combination of 3 elements: a NIR spectrometer, a sampling cartridge and the software (algorithm).

The NIR spectrometer is preferably a high precision portable spectrometer which operates under digital light processing (DLP) technology. It preferably works on a reflectance setup that includes two broadband tungsten lamps to properly illuminate the sample and an input slit, collimating and focusing lenses that collect the optical information in the wavelength range from 900 nm to 1700 nm. During a scan, the sample placed against the sensor window absorbs a specific extent of NIR light and diffusely reflects the non-absorbed light into the collection lens. The amount of light that is absorbed at each wavelength is dependent on the molecular makeup of the sample and is specific for each material, which constitutes a chemical fingerprint. For an accurate and reliable performance of the sensor, the sample (cartridge) should preferably be placed directly against the sapphire window of the spectrometer. Unwanted physical shifts for sample position could lead to low illumination and as a consequence, low reflectance of non-absorbed light would originate erratic results.

The disclosed cartridge is multilayer paper-based, enabling a homogeneous dispersion of fluids and a correct classification and quantification of biochemical parameters of different cellular blood components and/or biochemical metabolites thereof on NIR spectrometer. The disclosed cartridge is disposable and displays mechanical, chemical and optical properties that allow blood samples to uniformly spread in the cartridge for an accurate reading by the spectrometer.

The cartridge is preferably composed of five different layers:
Layer alpha: paper based layer wherein at least two types of paper are applied. Details are described further below.
Layer beta: an element that homogeneously reflects preferably at least 95% of NIR radiation, free of reagents, inert to the sample;
Layer gamma: a highly stable and compact support that allows the reproducibility of the cartridge and thus assures the precision of the obtained results;
Layer delta: a hydrophobic and protective layer to confine the sample into a specific area, which has an opening which contributes to confining the sample to a small area below the said opening, such that the sample will form a highly homogenous distribution in the specific measurement area;
Layer omega: a highly transmissive in the spectrometer's spectrum, optically clear layer to protect the cartridge and avoid moisture and contamination.

Additionally, the cartridge can be renewed according to the final application (analyte(s) of interest). This means that the definition of the layers and its architecture can depend on the type of fluid to analyze.

The software refers to a data processing module. This module preferably has several sub-processes which includes the following: save the recorded spectrum on a file, pre-process the file, process the spectrum with methods of spectral normalization and correlate for parameter quantification, preferably applying methods of variable selection and dimensionality reduction. The correlation methods are for example performed through multivariate regression, namely Partial Least Squares (PLS) or Support vector machines (SVM), or machine-learning techniques like Random decision forests. The final process exports the result value for each parameter.

Homogeneity of the fluid dispersion is important as a typical sensor imaging area is rather substantial and thus lack of homogeneity in the sensor area will be a source of error. Also, the overall reflectance of the cartridge assembly, mostly given by the combination of the paper-based alpha layer and the reflecting beta layer, is relevant for obtaining precise results. Also, the results have been found to be highly dependent on the physical stability of the assembled cartridge which is provided by a support gamma layer, ensuring that even as the liquid sample infuses the assembly, there is no appreciable bending, warping or curling of the assembly layers.

As mentioned above, layer alpha is a paper-based layer that includes two types of paper dependent on the application: a first paper type (type 1 paper) for separating and analysing different cellular blood components, and a second paper type (type 2 paper) for separating and analysing biochemical metabolites mostly derived from the cellular components of the blood, such as bilirrubin.

The different papers used as layer alpha are described in the following in more detail.

Type 1 paper for separating and quantifying cellular blood components requires a diffusely reflecting material that should be sufficiently porous in all directions to allow a liquid to spread uniformly. Homogeneity in the sample dispersion is fundamental for the validation and reproducibility of the method and can be achieved by choosing suitable physical-chemical characteristics of the alpha layer. Together with pore size and thickness, flow rate is directly influenced by the membrane porosity and its performance affects straightforwardly the analyte deposition, the sensitivity and the specificity of the paper. Therefore, the preferred features for this application of Type 1 paper are:
- pore size: between 6 and 10 µm, preferably between 8 and 10 µm;
- porosity: homogeneous porosity / pore size distribution along the alpha layer;
- thickness: between 150 and 400 µm, preferably between 180 and 390 µm, more preferably between 200 and 300 µm.

The basis weight of Type 1 paper is between 80 and 200 g/m2, preferably between 84 and 187 g/m2, more preferably between 88 and 180 g/m2. The required volume of a blood sample to be applied to the type 1 paper for quantification is between 10 and 20 µl. Tensile strength is between 7-20 lb/in MD. The micron retention time is between 5 and 20 µm, preferably between 6 and 10 µm.

In one specific embodiment of the Type 1 layer, the paper of the paper-based layer is paper with a basis weight of 87.8 g/m², a thickness of 0.24 mm, a rapidity of 200 mls/min, a Frazier permeability of 36.0 L/m²/sec, a Tensile Strength MDT Dry of 2680 N/m, and a wet burst of 76.2 cm.

Type 2 paper for separating and quantifying biochemical analytes, such as biochemical metabolites of cellular components, has a thickness between 300 and 500 µm, preferably between 300 and 400 µm. The pore size is between 2 - 10 µm, preferably between 6 - 10 µm, more preferably between 8 - 10 µm, or preferably about 2.8 µm. The basis weight of Type 2 paper is between 70 and 250 g/m2, preferably between 70 and 233 g/m2, more preferably between 73 and 200 g/m2. The required volume of a blood sample to be applied to the type 2 paper for quantification is between 40 and 60 µl, such as 40 µl. Tensile strength is between 3-10 lb/in MD, preferably between 3.5 and 7.5, more preferably between 3.8 and 4.5. The micron retention time is between 2 and 5 µm, preferably between 2.8 and 3.2 µm.

In one specific embodiment of the Type 2 layer, the paper of the paper-based layer is paper with a basis weight of 73 g/m², a thickness of 0.33 mm, a time to absorb 100 µL of blood of 66s, a diameter of red blood cell migration for 100 µL of blood of 24 mm, an average volume of plasma extracted from 100 µL of blood of 12 µL, and a wicking rate s/4 cm of 74.

In yet a further embodiment the paper layer used as alpha layer may have a specific contact angle. The contact angle can be determined by static contact angle measurements using the sessile drop method in conjunction with a high performance image processing system. When applying this method a contact angle between 60 and 80°, preferably between 65 and 75°, such as 70° or 80° for gylcerol and a contact angle between 20 and 40°, preferably between 25 and 35°, such as 29° or 24° for diiodomethane were determined.

Also, the support gamma layer ensures physical stability and that compaction of the lower layers is obtained. The support gamma layer also provides a backing that ensures that the sample is pushed against the spectrometer window. The omega layer seals and closes against the other layers, in particular keeping the remaining layers between the omega and gamma layers, avoiding the formation of air bubbles.

Also, by confining the sample to a specific area by the delta layer, the liquid does not spread over a large area outside the measurement area of the spectrometer and is much more likely to infuse with homogeneity the specific measurement area. The total volume of absorbable sample is defined by the thickness of the paper layer and the area of the paper layer which is not obstructed by the delta layer. Also, by having a predetermined total volume of absorbable sample, there is no possibility of over-soaking the cartridge with liquid and thus impeding the spectrometer measurement. These features improve the precision and repeatability of the spectrometer measurements. The delta layer may be a monomeric calendered PVC film 80 microns thick with acrylic solvent-based adhesive with permanent adhesion.

It is hereby disclosed a cartridge for a near-infrared, NIR, spectroscopic device for analyte quantification in a sample of a biological fluid, comprising a multilayered product comprising a porous paper-based layer for absorbing and spreading the sample in substantially all of a predetermined area at a front side of the paper-based layer for the spectroscopic device to measure.

In an embodiment, said paper-based layer comprises a NIR diffusely-reflecting paper material of uniform thickness which provides a uniform optical path across said predetermined area.

In an embodiment, the multilayered product further comprises a reflective layer for reflecting NIR light and backing the paper-based layer.

In an embodiment, said reflective layer is homogenously reflective of NIR light across said predetermined area.

In an embodiment, said reflective layer is reflective of at least 95% of NIR light.

In an embodiment, the multilayered product further comprises a back-support layer to stabilize the paper-based layer and, if existing, the reflective layer.

In an embodiment, the multilayered product further comprises a protective layer of the front side of the paper-based layer, said protective layer being hydrophobic for containing the absorbing and spreading of the sample to the predetermined area.

In an embodiment, the protective layer has an opening which matches the predetermined area for containing the absorbing and spreading of the sample to said predetermined area.

In an embodiment, the paper-based layer has a thickness of 200-400 µm, preferably between 200-300 µm.

In an embodiment, the pore size of the paper-based layer is between 2 - 10 µm, preferably between 6 - 10 µm, more preferably between 8 - 10 µm, or preferably about 2.8 µm.

In an embodiment, the multilayered product further comprises a top cover layer which is liquid-tight.

In an embodiment, the top cover layer is fluid tight and in particular transparent.

It is also disclosed a kit for analyte quantification in a sample of a biological fluid comprising a near-infrared, NIR, spectroscopic device and one or more of the cartridge according to any of the previous claims.

It is also disclosed a method for manufacturing a cartridge for use by a near-infrared, NIR, spectroscopic device for analyte quantification in a sample of a biological fluid, comprising the steps of:
providing a multilayered product with a porous paper-based layer for absorbing and
spreading the sample in substantially all of a predetermined area at a front side of the paper-based layer for the spectroscopic device to measure.

In an embodiment, said paper-based layer comprises a NIR diffusely-reflecting paper material which provides a uniform optical path across said predetermined area.

An embodiment further comprises the step of providing the multilayered product with a reflective layer for reflecting NIR light and backing the paper-based layer.

In an embodiment, said reflective layer is homogenously reflective of NIR light across said predetermined area.

In an embodiment, said reflective layer is reflective of at least 95% of NIR light.

An embodiment further comprises the step of providing the multilayered product with a back-support layer to stabilise the paper-based layer and, if existing, the reflective layer.

An embodiment further comprises the step of providing the multilayered product with a protective layer of the front side of the paper-based layer, said protective layer being hydrophobic for containing the absorbing and spreading of the sample to the predetermined area.

In an embodiment, the protective layer has an opening which matches the predetermined area for containing the absorbing and spreading of the sample to said predetermined area.

An embodiment further comprises the step of providing the multilayered product with a top cover layer which is liquid-tight.

In an embodiment, the top cover layer is fluid tight and in particular transparent to NIR radiation.

### BRIEF DESCRIPTION OF THE DRAWINGS

The following figures provide preferred embodiments for illustrating the disclosure and should not be seen as limiting the scope of invention.
**Figure 1****:** Results for an embodiment with a PLS correlation for erythrocytes (a), hemoglobin (b) and hematocrit (c) performed with 10 blood samples placed in the cartridge using Whatman Grade 3 Qualitative Filter Paper Standard Grade", i.e. grade 3, according to the disclosure.
**Figure 2****:** Results for an embodiment with a PLS correlation for erythrocytes (a), hemoglobin (b) and hematocrit (c) performed with the same 10 blood samples of figure 1, placed in the same paper of cartridge, but between glass slides.
**Figure 3****:** Results for an embodiment with a PLS correlation for erythrocytes (a), hemoglobin (b) and hematocrit (c) performed with the same 10 blood samples of figure 1 and 2, placed in the cartridge with a different alpha layer, using "Ahlstrom Cytosep 1660 Plasma Separation Media", i.e. grade 1660.

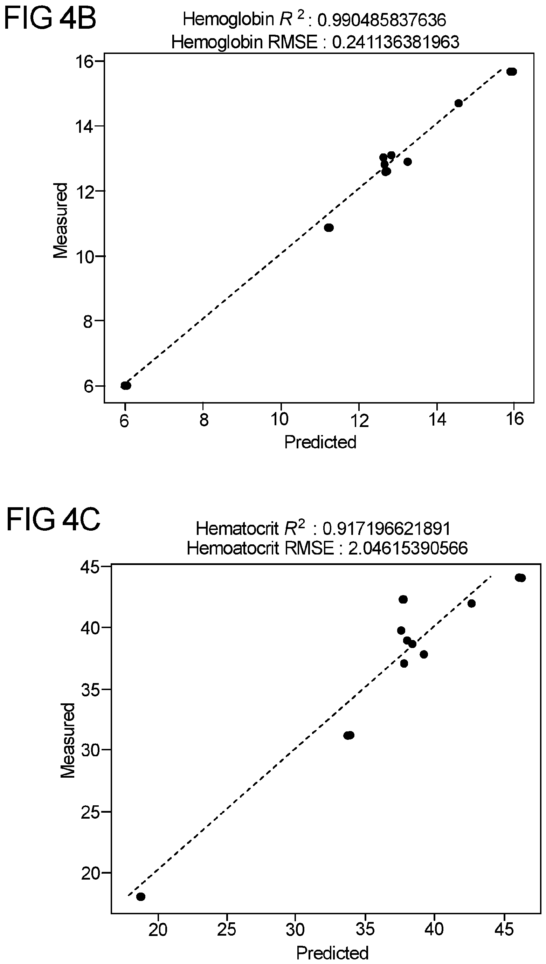

**Figure 4****:** Results for an embodiment with PLS correlation for erythrocytes (a), haemoglobin (b) and hematocrit (c) performed with the same 10 blood samples of figure 1, 2 and 3, placed in the cartridge with a different alpha layer, using "Ahlstrom Grade 631 Qualitative Filter Paper Standard Grade", i.e. grade 631.
**Figure 5****:** Schematic representation of an embodiment of a NIR spectrometer.
**Figure 6****:** Schematic representation of an embodiment of a cartridge: a) Front-view of the cartridge; b) Detailed view of the cartridge with measures (in mm); c) Orthographic view; d) Exploded view of the cartridge with layers: 1- layer omega; 2- layer delta; 3-layer alpha; 4- layer beta; 5- layer gamma.
**Figure 7****:** Schematic representation of an embodiment of spectroscopic platform: a) Spectrometer embodiment; b) Cartridge embodiment; c) Cartridge embodiment assembly; d) Junction of both embodiments; e) Cartridge embodiment sliding on spectrometer embodiment; f) Final configuration ready for analysis.
**Figure 8****:** Optional design of an embodiment for blood sample support: Paper-based material between glass slides: a) orthographic view; b) lateral view with: 1- glass slides; 2- paper-based material.
**Figure 9****:** Schematic representation of an embodiment of a spectrometer optical architecture: sample (a), reflectance module (b), the optical engine (c) and the generated output, spectrum (d).

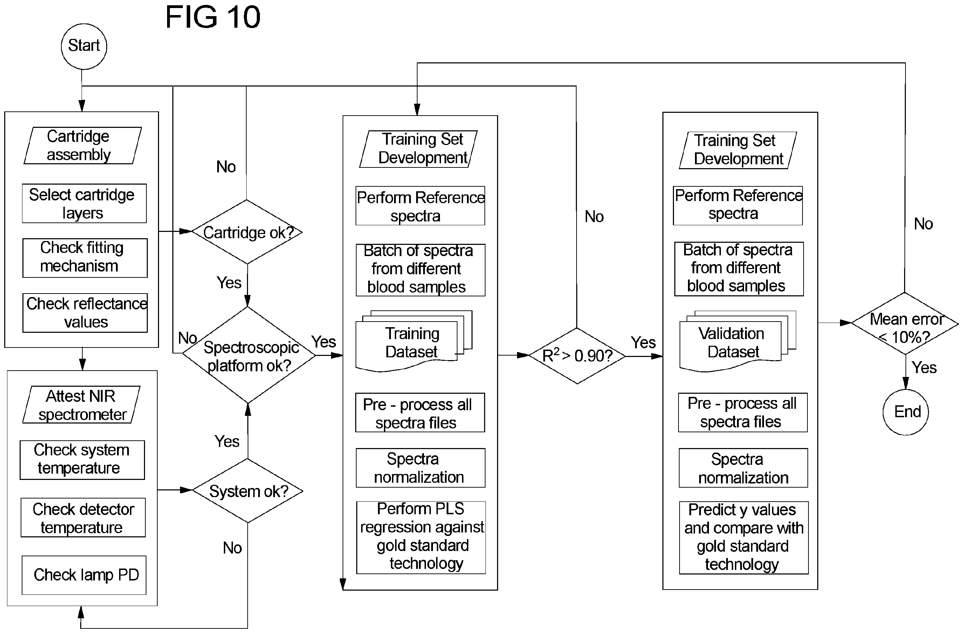

**Figure 10****:** Flowchart of operation according to an embodiment of the disclosure.

### DETAILED DESCRIPTION

As described above, the present disclosure includes the combination of 3 elements: a NIR spectrometer, a sampling cartridge and the software (algorithm).

Said three elements are used in a method for quantifying an analyte in a biological fluid, in particular in a sample of a blood. The method comprises the steps of taking a sample of a biological fluid, placing the sample in a cartridge as described in detail above and placing the cartridge in at least one NIR spectrometer; recording at least one NIR-spectrum of said sample and processing and analyzing the NIR spectrum taken of said sample.

### Specifically, the method comprises

- Providing a blank reference sample, for example by using a blank cartridge;
- Providing at least one sample of a biological fluid in the cartridge;
- Recording at least one NIR spectrum of the sample of the biological fluid; in particular recording multiple spectra for each sample (minimum 3);
- Applying at least one digital filter to the NIR spectrum obtained for removing electronic noise and diffraction artifacts, in particular the Standard Normal Variates (SNV);
- Reducing the number of variables for those that maximize the correlation with the real value of the sample through the application of multivariate analysis algorithms, such as PLS or similar; and
- Algebraic manipulation of the values obtained and calculation of the concentration of the different metabolites using the embedded models.

Thus, a digital processing is applied that transforms the spectral data by digital filtering in 3 to 5 stages on a given sequence and subsequently a standard algebra process is applied to the filtered data.

As described above, PLS regression with cross-validation can be preferably used to compare the predictive ability as a function of the number of latent variables included. R² is a statistical measure of how well a regression line approximates the real data points and an R² equal to 1 represents a perfect fit. Normalized absorbance spectra obtained is correlated with gold-standard values provided by Siemens ADVIA 120 Hematology System.

The system is optimized for the collection of 20µL of blood and posterior dispersion in the cartridge with the alpha layer of Whatman 3. Volume variations of 10µL do not constitute significant output errors, so the system works in finger-prick blood collection without the need for a volumetric pipette.

The following step is to ensure homogeneous dispersion within a waiting time of 60 seconds. This is critical to ensure correct sample diffusion, stabilization and hence its analysis. Tests performed at 60 ± 30 seconds intervals produces significant errors in the analysis and do not allow a correct measurement. After 60 seconds, it is necessary to slide the omega layer to seal the cartridge and place it in the analysis position. If these 3 steps were guaranteed, the platform will correctly predict values for the parameters described in the patent summary, regardless of the person's gender / age / pathology.

Siemens ADVIA 120 Hematology System uses the gold standard technology that analyses the same samples as our platform; against which measurements in our platform were compared. The results exported by Siemens ADVIA represent the actual values of the sample (y) that will be computed in the linear regression algorithm, PLS.

Partial Least Squares (PLS) is a supervised technique mainly used for quantification analysis. Its principal aim is to find the latent variables that describe the variance in the data and also reach the maximum correlation between predictive and predictor variables. PLS is used to extract factors related to one or more response values, and its validation was performed using the cross-validation method. PLS regression with cross-validation aims to compare the predictive ability as a function of the number of components included. The smaller the number of components used in the algorithm, the greater its reliability when applied. The models used in this patent have only 1 component and are therefore considered robust models.

The data acquired on our platform undergoes a normalization process (spectral preprocessing) before being entered into the PLS algorithm. This spectral preprocessing is very important in biophotonic systems like NIR spectral signals, particularly those that are associated with light scattering variations; these are responsible for additive and multiplicative effects that make linear calibration of NIR diffuse reflectance more difficult. The correction of the additive and multiplicative effects aims to reduce physical noise and offer the same weight to each signal, and it can be done by applying the Standard Normal Variates (SNV) transformation. With this method, each signal is centered and divided by its standard deviation.

This method corrects potential artefacts presented in the raw spectra and tries to compensate for some electronic and physical noise with signal correction strategies. Correlation scores attest the value of normalization methods when comparing PLS performance in raw and normalized spectra. In our application, SNV is the best spectral pre-processing method that maximizes correlation in the PLS algorithm. After normalization, PLS is applied between the normalized NIR spectrum data (x) and the values measured by the gold standard (y).

The algorithm exports a model (with a B vector and a B0 value) which is measured in terms of with R² and RMSE. R² is a statistical measure of how well a regression line approximates the real data points; an R² equal to 1 represents a perfect fit. RMSE is defined as the Root Mean Square Error. It is expressed as an average modelling error in the same units as the original response values.

B vector and B0 will then be used to predict a new y, based on normalized measured x.

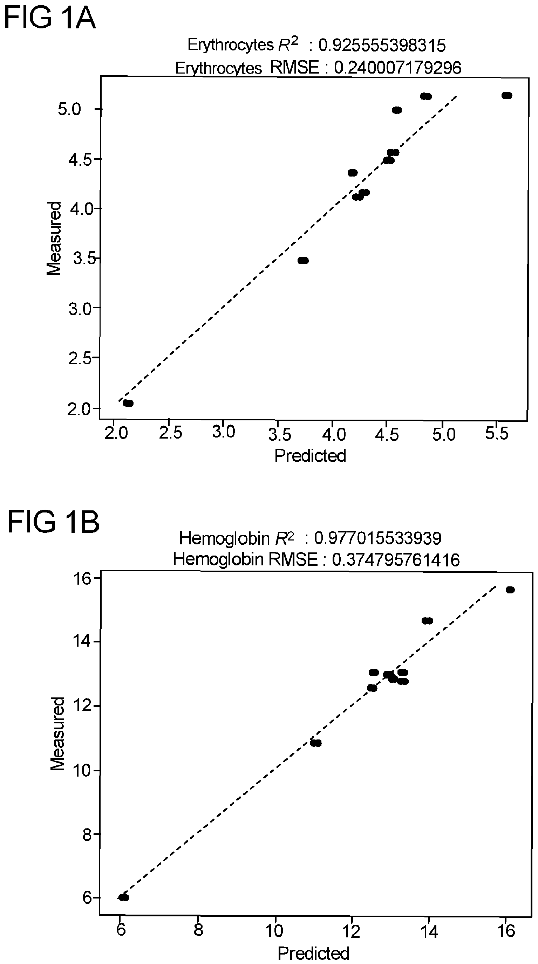

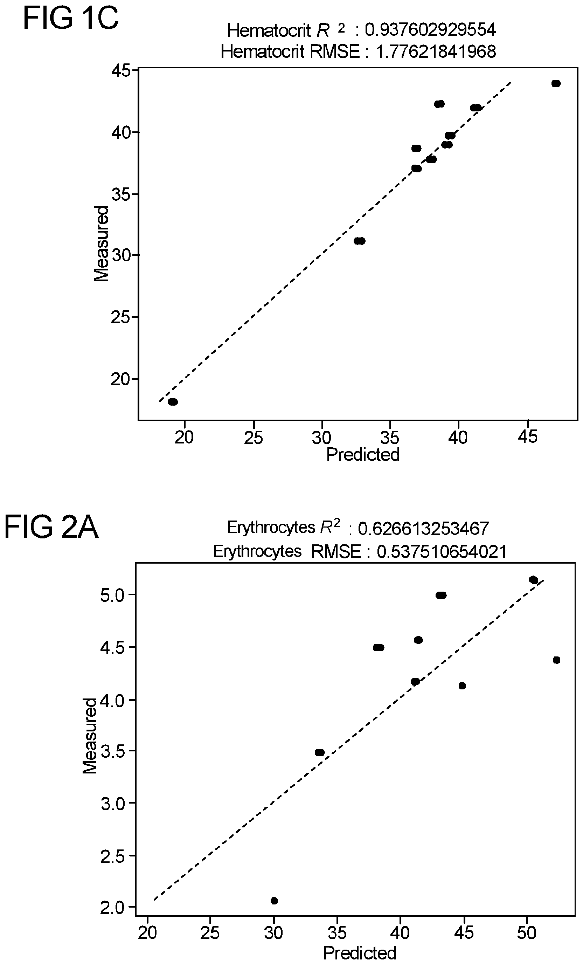

**Figure 1** presents the correlation plot of 10 blood samples which were processed in the proposed platform (NIR spectrometer + cartridge).

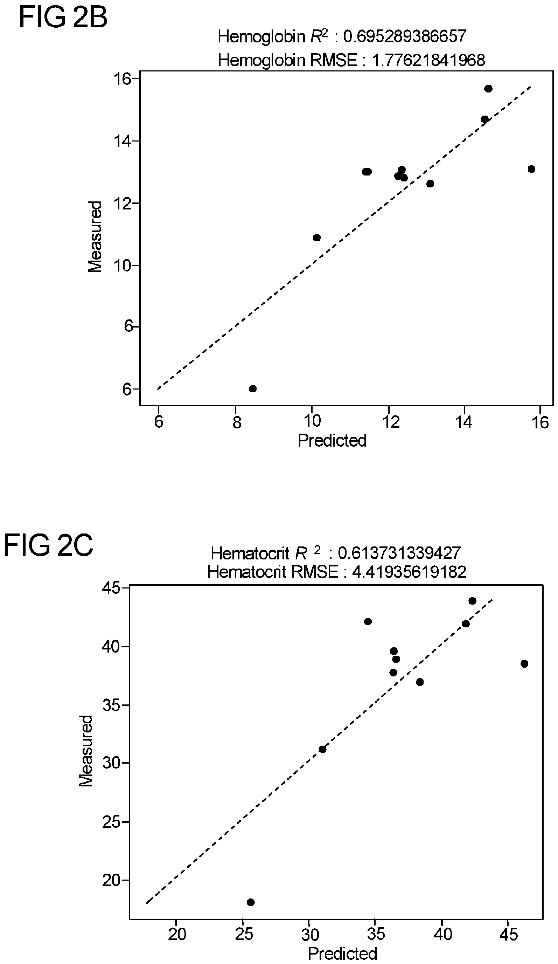

The same samples were also processed in a different configuration to compare the efficiency of the cartridge with alpha, beta, gamma, delta and omega layers. In this second approach, **Figure 2****,** the blood samples were presented to the spectrometer in a paper-based material (the same of cartridge), but between glass slides.

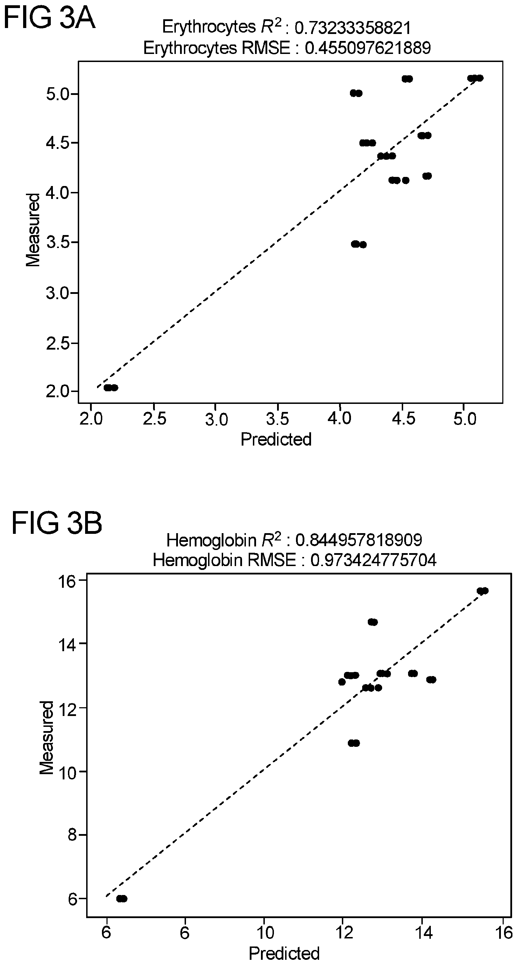

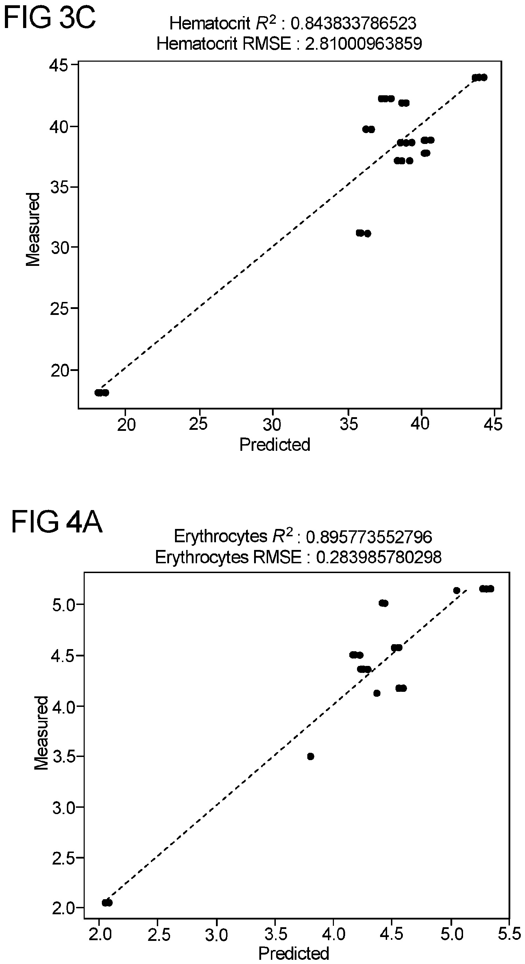

An additional test was accomplished to test the alpha layer. A different paper-based grade was applied, with different mechanical, chemical and optical properties. The PLS regression was tested as well as previous tests and is plotted in **Figure 3****.**

A potential alternative, which was tested and is represented in the results of figure 3, encompasses the change of the alpha layer of the cartridge by another paper-based material that can offer different mechanical, chemical and optical characteristics for a given fluid.

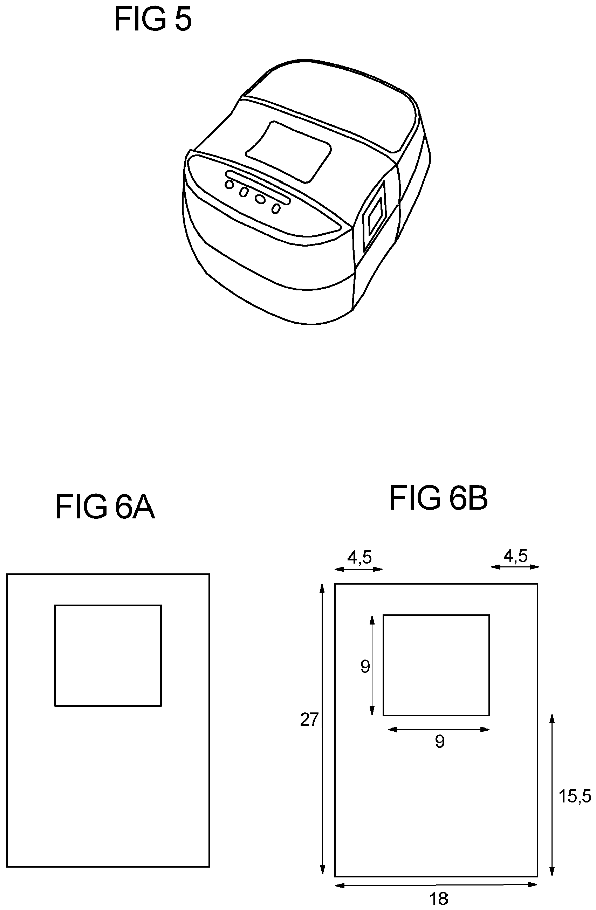

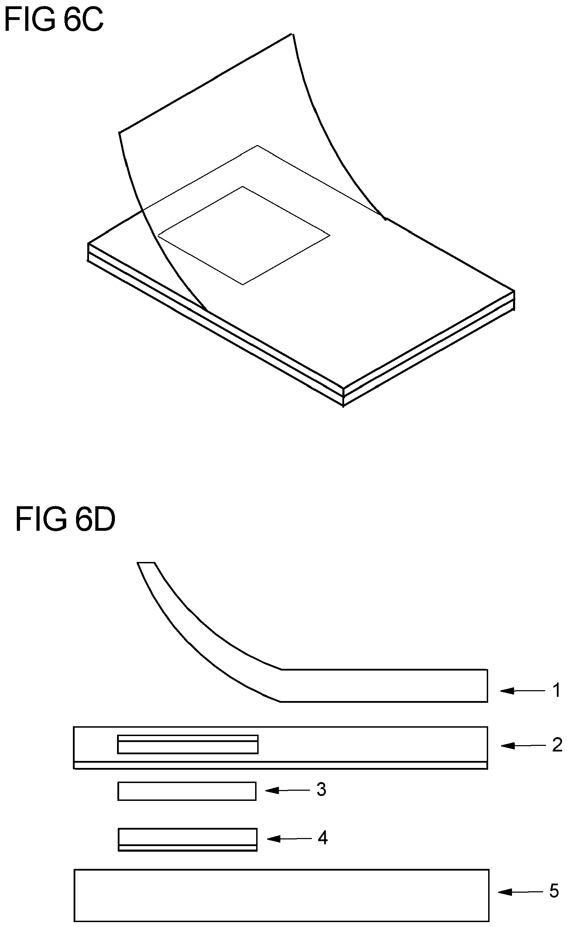

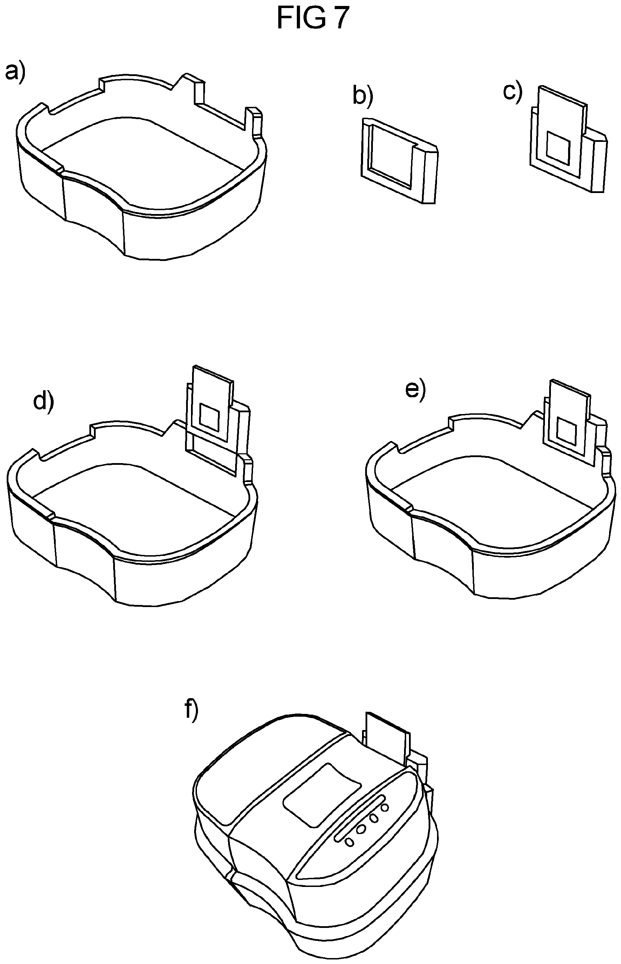

**Figure 5** shows a NIR spectrometer. **Figure 6** shows an embodiment of a cartridge: a) Front-view of the cartridge; b) Detailed view of the cartridge with measures (in mm); c) Orthographic view; d) Exploded view of the cartridge with layers: 1- layer omega; 2-layer delta; 3- layer alpha; 4- layer beta; 5- layer gamma. **Figure 7** shows a schematic representation of spectroscopic platform: a) Spectrometer embodiment; b) Cartridge embodiment; c) Cartridge embodiment assembly; d) Junction of both embodiments; e) Cartridge embodiment sliding on spectrometer embodiment; f) Final configuration ready for analysis.

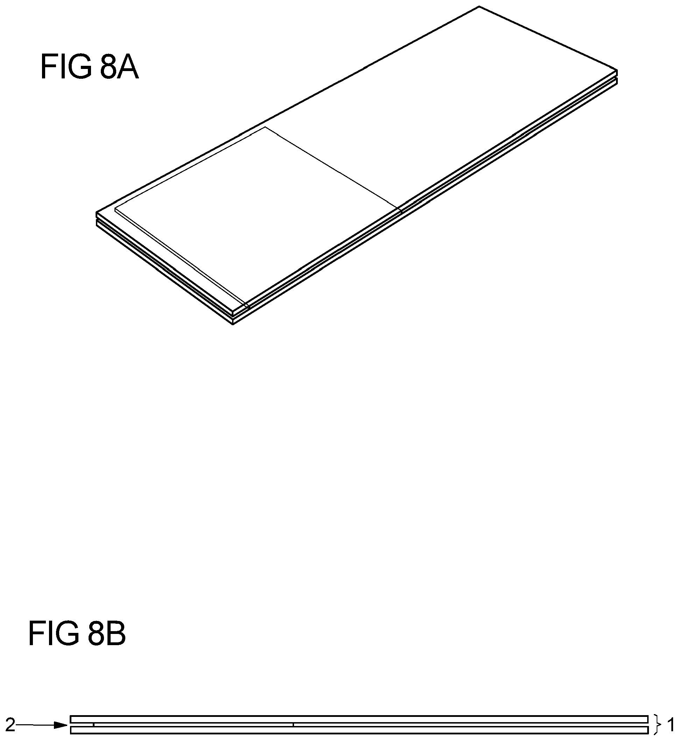

The reference example depicted in **Figure 8** concerns the use of two glass slides which guarantee that the sensor remains clean and free of contaminations while providing physical support to the paper matrix. However, this configuration was not always reproducible over time and can become a source of reading errors that can be solved by sample holder modifications as explained below. Figure 8 shows an optional design for blood sample support: Paper-based material between glass slides: a) orthographic view; b) lateral view with: 1- glass slides; 2- paper-based material.

The disclosure is portable, non-invasive, providing a point-of-care diagnostic platform with a disposable cartridge, also being reagents-free and consumables-free. The disclosure works with low sample volume (< 30 µL) and executes multi-analyte detection with one blood sample. The disclosure performs classification of pathological states and quantification of biological parameters, specially hemoglobin concentration and erythrocytes and hematocrit counts. The disclosure does not require health qualified professionals to perform a test or user intervention during the analysis steps. It produces real-time, automatic results that do not require interpretation or calculations, validated against the gold standard technology, namely Siemens ADVIA 120 Hematology System.

As explained above, the spectrometer was found to be highly sensitive to certain features that preferably need to be valued in order to achieve maximum system performance. For an accurate and reliable performance of the sensor, the sample (cartridge) should preferably be placed directly against the sapphire window of the spectrometer. Physical shifts for sample position could lead to low illumination and as a consequence, low reflectance of non-absorbed light would originate erratic results (systematic errors). The support layer is also important to achieve this physical stability and contact with the spectrometer window. The advantage in excluding these systematic errors is provided by the development of an embodiment for the spectrometer, which is able to stabilize and ensure the reproducibility of the system.

For sample (cartridge) presentation to the spectrometer, the embodiment was redesigned with a vertical fit mechanism adapted to the cartridge. This mechanism was achieved by structuring a V-matrix (see Figure 7, the V-matrix corresponds to the fitting mechanism design of the spectrometer embodiment) in the spectrometer embodiment and a "negative" or complementary V-matrix in the cartridge embodiment (complementary V-Matrix relates to the fitting mechanism design of the cartridge embodiment), which allowed a second-level of stabilization, not only in the spectrometer, but also in sample presentation and sample analysis. Several iterations were made for proper assembly in the interface of both structures (spectrometer and cartridge). A best compaction solution, which means the absence of space in x axis, was obtained.

The multi-layer, paper-based cartridge construction was built with the combination of three aspects: mechanical, optical and chemical properties. From a mechanical point of view, stability principle of the spectrometer was also employed in the cartridge with the help of layer gamma. The cartridge (all assembled) preferably have to reflect more than 95% of NIR radiation, otherwise reflections would not be collected into the spectrometer with quality, or alternatively would be collected with low intensity which would lead to a low correlation factor. For this reason, layer beta was employed.

An important parameter in the provision of the cartridge is the choice of the paper-based material (alpha layer), which depends in particular on the type of analysis (cellular components or biochemical metabolites), but also on several other factors, namely the combination of the physical and chemical characteristics of the sample and the material itself. For example when using type 1 paper for separating and analysing cellular components, pore size, pore size distribution, thickness of the paper layer may have to be selected depending on the sample volume. For example, when analysing blood samples of about 20 µl (for example in case of venous blood preserved in EDTA tubes) a suitable paper layer should preferably have a pore size between 6 and 10 microns and a thickness between 200 and 400 microns. When analysing blood samples of about 10 µl (for example for blood collected in a finger prick method) a suitable alpha paper layer must have other properties, namely in capillary flow rate, which is highly dependent on pore size, pore size distribution and thickness. The capillary flow rate corresponds to the speed of the sample front along a paper strip and its performance affects straightforwardly the analyte deposition, the sensitivity and the specificity of the membrane. Suitable paper layers are for example Ahlstrom Grade 1 and Ahlstrom Grade 631 papers (see also below).

Figure 3 shows an example of a different paper in the correlation factor for 10 blood samples, when compared to figure 1. These properties disclosed above may be adapted by the skilled person as necessary, according the present disclosure, in order to achieve the results herein described.

A cartridge according to the disclosure may have a paper-based layer of Whatman Grade 3 Qualitative Filter Paper Standard Grade (tm), Ahlstrom Cytosep 1660 Plasma Separation Media (tm), or Ahlstrom Grade 631 Qualitative Filter Paper Standard Grade (tm), or combinations thereof.

For example, the paper-based layer may have a paper with a basis weight of 73 g/m2, a thickness of 0.33 mm, a time to absorb 100 µL of blood of 66s, a diameter of red blood cell migration for 100 µL of blood of 24 mm, an average volume of plasma extracted from 100 µL of blood of 12 µL, and a wicking rate s/4 cm of 74.

For example, the paper-based layer may have a paper with a basis weight of 87.8 g/m2, a thickness of 0.24 mm, a rapidity of 200 mls/min, a Frazier permeability of 36.0 L/m²/sec, a Tensile Strength MDT Dry of 2680 N/m, and a wet burst of 76.2 cm.

The contact angel of the paper-based layer was determined as follows: Static contact angle measurements were obtained using the sessile drop method and an OCA 15plus goniometer with a high-performance image processing system (DataPhysics Instruments, Germany). 3µL of either Glycerol or Diiodomethane were added using a motor driven syringe at a rate of 4µL/s, room temperature by using at least five measurements per condition. The values of Surface Energy were calculated by the Owens, Wendt, Rabel and Kaelble (OWRK) method using software SCA20 version 2. The following values for each liquid were used in these calculations: **Glycerol:** Surface tension: 62.70 mN/m; Dispersive component: 21.20 mN/m; Polar component: 41.50 mN/m

**Diiodomethane:** Surface tension: 50.0 mN/m; Dispersive component: 47.40 mN/m; Polar component: 2.60 mN/m

### Results:

| Sample | Contact Angle (º) | | Surface Energy (mN/m) | | |
|---|---|---|---|---|---|
| | | | Surface Energy | Dispersive component | Polar component |
| Whatman grade qualitative 3 | Glycerol | 69.78 ± 5.42 | 43.28 ± 0.02 | 38.56 ± 0.02 | 4.72 ± 0.00 |
| | Diiodomethane | 29.36 ± 1.95 | | | |
| Ahlstrom grade qualitative 631 | Glycerol | 79.03 ± 5.73 | 43.68 ± 0.02 | 42.06 ± 0.02 | 1.61 ± 0.00 |
| | Diiodomethane | 24.13 ± 1.14 | | | |
| I.W. Tremont CF-D23-TC1 | Glycerol | 124.94 ± 9.33 | 57.88 ± 0.03 | 48.69 ± 0.03 | 9.19 ± 0.00 |
| | Diiodomethane | 47.98 ± 3.63 | | | |
| I.W. Tremont LF-D23-TC1 | Glycerol | 133.08 ± 6.33 | | | |
| | Diiodomethane | < 20 | | | |

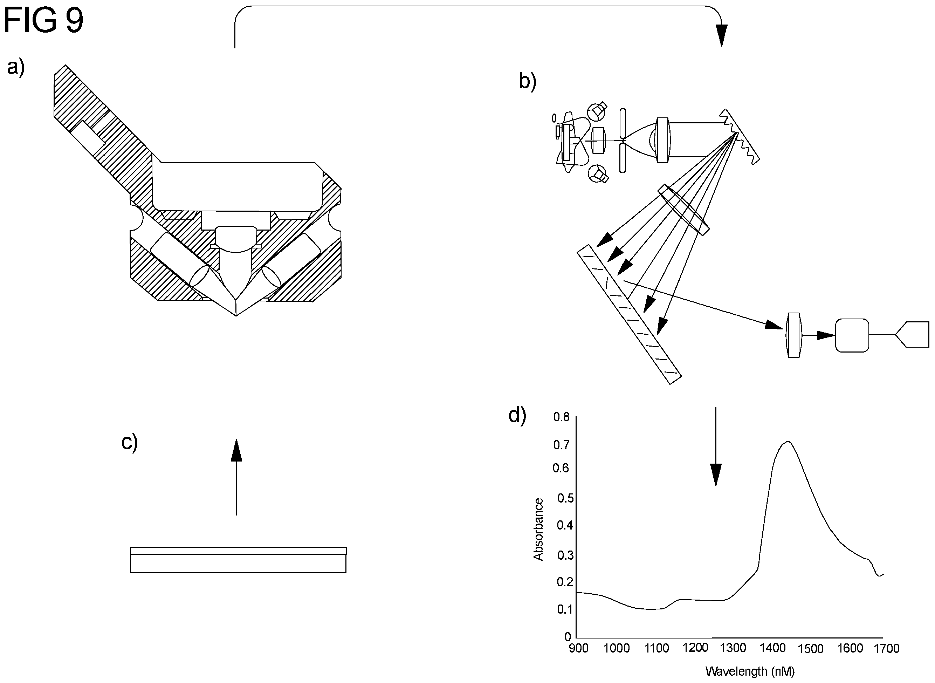

**Figure 9** shows a spectrometer optical architecture with: sample (a), reflectance module (b), the optical engine (c) and the generated output, spectrum (d).

The NIR spectrometer is preferably a high precision portable spectrometer which operates under digital light processing (DLP) technology. It preferably works on a reflectance setup that includes two broadband tungsten lamps to properly illuminate the sample and an input slit, collimating and focusing lenses that collect the optical information in the wavelength range from 900 nm to 1700 nm. During a scan, the sample placed against the sensor window absorbs a specific extent of NIR light and diffusely reflects the non-absorbed light into the collection lens. The amount of light that is absorbed at each wavelength is dependent on the molecular makeup of the sample and is specific for each material, which constitutes a chemical fingerprint. For an accurate and reliable performance of the sensor, the sample (cartridge) should preferably be placed directly against the sapphire window of the spectrometer.

Spectroscopy can be defined as the study of the interaction of electromagnetic radiation with matter. Molecular absorption technique that gives rise to vibration and molecular rotation phenomena visible in spectrum. This technique obeys to Beer Law which states that the measured absorbance is a function of the concentration of the analyte (s) present in the sample.

The spectrometer preferably supports a wavelength range from 900 to 1700 nm and may apply a preprocessing comprising a Hadamard transform which is an example of a generalized class of Fourier transforms. The Hadamard preprocessing offers greater signal to noise ratio. Therefore, it is considered a preferred method for pattern creation and recognition.

The sensor preferably has an optical resolution of 10 to 12 nm and should preferably be operated on a temperature range between 0 and 50 ºC.

This spectrometer preferably uses a 25 µm wide by 1.8 mm tall slit. The light that passes through the slit is collimated by the first set of lenses, passes through a filter, preferably an 885 nm long wavepass filter, and then strikes a reflective grating. This grating, in combination with the focusing lens, disperses the light into its constituent wavelengths. The focusing lenses form an image of the slit at the digital micromirror device (DMD). The optical system preferably images 900-nm wavelengths to one end of the DMD and 1700-nm to the other end, with all other wavelengths sequentially dispersed in between. When specific detector columns are selected as *on,* or tilted to the +17° position, the energy reflected by the selected columns is directed through the collection optics to the single pixel, preferably InGaAs, detector.

**Table 1: Working specifications of the spectrometer.**

| **PARAMETER** | **MIN** | **TYP** | **MAX** | **UNIT** |
|---|---|---|---|---|
| Supported wavelengths | 900 | | 1700 | nm |
| Optical resolution | | 10 | 12 | nm |
| Lamp power | | 1.4 | | W |
| Temperature⁽¹⁾ | 0 | 25 | 50 | °C |

**Table 2: Spectrometer scan configuration parameters.**

| **Scan Configuration Parameters** | **20-nm Content** | **15-nm Content** | **10-nm Content** | **8-nm Content** | |
|---|---|---|---|---|---|
| Wavelength range | 900 to 1700 nm | 900 to 1700 nm | 900 to 1700 nm | 900 to 1700 nm | 900 to 1700 nm |
| Width in nm | 20 | 15 | 10 | 8 | 8 |
| Digital Resolution | 80 | 108 | 160 | 225 | 248 |
| Oversampling | 2 | 2 | 2 | 2.25 | 2.48 |
| Number of scans to average | 18 | 12 | 8 to 9 | 6 | 5 |

For each scan, the system preferably saves: the method performed in the scan (optionally with Hadamard preprocessing), date and time, shift vector coefficients, system header version, serial number, system temperature, detector temperature, humidity, lamp photodetector number, the scan configuration type, exposure time, pattern pixel width, digital resolution, number of scans per average, total measurement time and the absorbance data for each wavelength.

The term "comprising" whenever used in this document is intended to indicate the presence of stated features, integers, steps, components, but not to preclude the presence or addition of one or more other features, integers, steps, components or groups thereof.

Flow diagrams of particular embodiments of the presently disclosed methods are depicted in figures. The flow diagrams illustrate the functional information one of ordinary skill in the art requires to perform said methods required in accordance with the present disclosure.

It will be appreciated by those of ordinary skill in the art that unless otherwise indicated herein, the particular sequence of steps described is illustrative only and can be varied without departing from the disclosure. Thus, unless otherwise stated the steps described are so unordered meaning that, when possible, the steps can be performed in any convenient or desirable order.

It is to be appreciated that certain embodiments of the disclosure as described herein may be incorporated as code (e.g., a software algorithm or program) residing in firmware and/or on computer useable medium having control logic for enabling execution on a computer system having a computer processor, such as any of the servers described herein. Such a computer system typically includes memory storage configured to provide output from execution of the code which configures a processor in accordance with the execution. The code can be arranged as firmware or software, and can be organized as a set of modules, including the various modules and algorithms described herein, such as discrete code modules, function calls, procedure calls or objects in an object-oriented programming environment. If implemented using modules, the code can comprise a single module or a plurality of modules that operate in cooperation with one another to configure the machine in which it is executed to perform the associated functions, as described herein.

The disclosure should not be seen in any way restricted to the embodiments described and a person with ordinary skill in the art will foresee many possibilities to modifications thereof. The above described embodiments are combinable. The following claims further set out particular embodiments of the disclosure.

## Claims

1. Cartridge for a near-infrared, NIR, spectroscopic device for analyte quantification in a sample of a biological fluid, comprising a multilayered product comprising a porous paper-based layer for absorbing and spreading the sample in substantially all of a predetermined area at a front side of the paper-based layer for the spectroscopic device to measure.

2. Cartridge according to the previous claim wherein said paper-based layer comprises a NIR diffusely-reflecting paper material of uniform thickness which provides a uniform optical path across said predetermined area.

3. Cartridge according to any of the previous claims wherein the multilayered product further comprises a reflective layer for reflecting NIR light and backing the paper-based layer.

4. Cartridge according to the previous claim wherein said reflective layer is homogenously reflective of NIR light across said predetermined area, in particular of at least 95% of NIR light..

5. Cartridge according to any of the previous claims wherein the multilayered product further comprises a back-support layer to stabilize the paper-based layer and, if existing, the reflective layer.

6. Cartridge according to any of the previous claims wherein the multilayered product further comprises a protective layer of the front side of the paper-based layer, said protective layer being hydrophobic for containing the absorbing and spreading of the sample to the predetermined area.

7. Cartridge according to any of the previous claims wherein the paper-based layer has a thickness of 200-400 µm, preferably between 200-300 µm.

8. Cartridge according to any of the previous claims wherein the pore size of the paper-based layer is between 2 - 10 µm, preferably between 6 - 10 µm, more preferably between 8 - 10 µm, or preferably about 2.8 µm.

9. Cartridge according to any of the previous claims wherein the multilayered product further comprises a top cover layer which is liquid-tight, preferably fluid tight and in particular transparent.

10. Cartridge according to the previous claim wherein the top cover layer is fluid tight and in particular transparent.

11. Cartridge according to any of the previous claims wherein the paper of the paper-based layer is paper with a basis weight of 73 g/m², a thickness of 0.33 mm, a time to absorb 100 µL of blood of 66s, a diameter of red blood cell migration for 100 µL of blood of 24 mm, an average volume of plasma extracted from 100 µL of blood of 12 µL, and a wicking rate s/4 cm of 74.

12. Cartridge according to any of the claims 1-12 wherein the paper of the paper-based layer is paper with a basis weight of 87.8 g/m², a thickness of 0.24 mm, a rapidity of 200 mls/min, a Frazier permeability of 36.0 L/m²/sec, a Tensile Strength MDT Dry of 2680 N/m, and a wet burst of 76.2 cm.

13. Kit for analyte quantification in a sample of a biological fluid comprising a near-infrared, NIR, spectroscopic device and one or more of the cartridge according to any of the previous claims.

14. Method for quantifying an analyte in a biological fluid, in particular in a sample of a blood, the method comprising the steps of taking a sample of a biological fluid, placing the sample in a cartridge as described in one of the claims 1-12 and placing the cartridge in at least one NIR spectrometer; recording at least one NIR-spectrum of said sample and processing and analyzing the NIR spectrum taken of said sample.

15. Method according to claim , wherein the method comprises
- Providing a blank reference sample, for example by using a blank cartridge;
- Providing at least one sample of a biological fluid in the cartridge;
- Recording at least one NIR spectrum of the sample of the biological fluid; in particular recording multiple spectra for each sample (minimum 3);
- Applying at least one digital filter to the NIR spectrum obtained for removing electronic noise and diffraction artifacts, in particular the Standard Normal Variates (SNV);
- Reducing the number of variables for those that maximize the correlation with the real value of the sample through the application of multivariate analysis algorithms, such as PLS or 4similar; and
- Algebraic manipulation of the values obtained and calculation of the concentration of the different metabolites using the embedded models.
